(19) Europäisches Patentamt
European Patent Office
Office européen des brevets

(11) Veröffentlichungsnummer: **0 110 309**
**B1**

(12) **EUROPÄISCHE PATENTSCHRIFT**

(45) Veröffentlichungstag der Patentschrift: **19.02.86**

(51) Int. Cl.⁴: **C 07 C 69/16,** C 07 C 67/00

(21) Anmeldenummer: **83111703.1**

(22) Anmeldetag: **23.11.83**

(54) **Verfahren zur Herstellung von 1,4-Diacyloxy-1,3-butadienen.**

(30) Priorität: **01.12.82 DE 3244369**

(43) Veröffentlichungstag der Anmeldung: **13.06.84 Patentblatt 84/24**

(45) Bekanntmachung des Hinweises auf die Patenterteilung: **19.02.86 Patentblatt 86/8**

(84) Benannte Vertragsstaaten: **BE CH DE FR GB IT LI**

(56) Entgegenhaltungen:
**DE - A - 695 865**
**DE - B - 1 020 621**

(73) Patentinhaber: **BASF Aktiengesellschaft, Carl-Bosch-Strasse 38, D-6700 Ludwigshafen (DE)**

(72) Erfinder: **Weitz, Hans-Martin, Dr., Auf dem Koeppel 40, D-6202 Bad Duerkheim (DE)**
Erfinder: **Fischer, Rolf, Dr., Bergstrasse 98, D-6900 Heidelberg (DE)**
Erfinder: **Paust, Joachim, Dr., Ringstrasse 3, D-6708 Neuhofen (DE)**

EP 0 110 309 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 1,4-Diacyloxy-1,3-butadienen, die in 2-Stellung durch Alkylreste substituiert sein können, durch Umsetzung entsprechender 4-Acyloxy-2-butenale mit Carbonsäureanhydriden in Gegenwart von Carbonsäuren und Salzen oder Pyridinderivaten.

Aus J. Org. Chem. *41*, 2627 (1976) ist bekannt, dass sich 2-Methyl-4-acetoxy-2-butenal mit Acetanhydrid in Gegenwart von Essigsäure und Kaliumacetat zu 2-Methyl-1,4-diacetoxy-1,3-butadien umsetzen lässt. Bei diesem Verfahren werden pro Mol 4-Acyloxy-2-butenal über 15 Mol Acetanhydrid angewendet. Man erhitzt das Reaktionsgemisch 19 Stunden lang unter Stickstoff auf 100 bis 115°C, fügt Chloroform hinzu und filtriert das ausgefällte Kaliumacetat ab. Durch Destillation des Filtrats erhält man ein Gemisch aus (E, E)-, (Z, E)- und (E, Z)-2-Methyl-1,4-diacetoxy-1,3-butadien in 81%iger Ausbeute. Auf entsprechende Weise kann man aus 4-Acetoxy-2-butenal 1,4-Diacetoxy-1,3-butadien herstellen [Chem. Ber. *90*, 192 (1957)]. Hierbei wird eine über 12fache molare Menge an Acetanhydrid eingesetzt, wobei man nach einer Behandlung des Reaktionsgemisches mit Ether und Destillation des Filtrates ein Gemisch aus (Z, Z)- und (Z, E)-1,4-Diacetoxy-1,3-butadien in 85%iger Ausbeute erhält.

Bei der technischen Ausübung dieses Verfahrens ergeben sich erhebliche Probleme, insbesondere hinsichtlich einer gewünschten störungsfreien Abtrennung der 1,4-Diacetoxy-1,3-butadiene aus dem Reaktionsgemisch. Hinzu kommt, dass ein Teil des Kaliumacetats in den Gemischen aus Chloroform bzw. Ether, Essigsäure und Acetanhydrid gelöst bleibt, wodurch die Destillation des 1,4-Diacetoxy-1,3-butadiens beeinträchtigt wird.

Es wurde nun gefunden, dass man bei der Herstellung von 1,4-Diacyloxy-1,3-butadienen der Formel

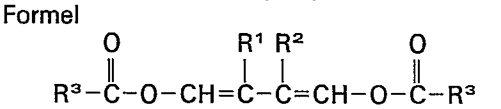

(I)

in der $R^1$ und $R^2$ Wasserstoff oder einer dieser Reste einen Alkylrest mit 1 bis 5 Kohlenstoffatomen und der andere Wasserstoff und $R^3$ einen Alkylrest mit 1 bis 15 Kohlenstoffatomen, einen cycloaliphatischen Rest mit 5 bis 7 Kohlenstoffatomen oder einen aromatischen Rest bedeuten, durch Umsetzung von 4-Acyloxy-2-butenalen der Formel

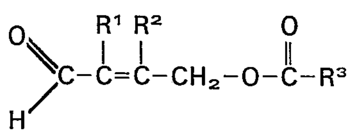

(II)

in der $R^1$, $R^2$ und $R^3$ die obengenannte Bedeutung besitzen, mit Carbonsäureanhydriden der Formel

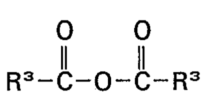

(III)

in Gegenwart der entsprechenden Carbonsäuren die genannten Nachteile vermeidet, wenn man, bezogen auf 1 Mol des 4-Acyloxy-2-butenals, weniger als 4 Mol des Carbonsäureanhydrids anwendet, die Umsetzung bei Temperaturen zwischen 0 und 200°C und entweder in Gegenwart von Ammonium- oder Alkalisalzen oder von Pyridinderivaten der Formel

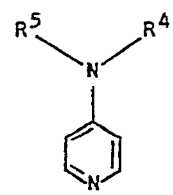

(IV)

in der $R^4$ und $R^5$ gleich oder verschieden sein können und Alkylreste mit 1 bis 5 Kohlenstoffatomen darstellen oder zusammen mit dem N-Atom einen Pyrrolidin-, Piperidin- oder Morpholinrest bedeuten, durchführt, nach der Umsetzung zum Reaktionsgemisch, bezogen auf ein Mol des eingesetzten 4-Acyloxy-2-butenals, 5 bis 100 Mol Wasser gibt und die 1,4-Diacyloxy-1,3-butadiene durch Abtrennung von der Wasserphase isoliert.

In den Ausgangsstoffen der Formel II stehen $R^1$ und $R^2$ vorzugsweise für ein Wasserstoffatom. Einer dieser Reste kann auch ein Alkylrest mit 1 bis 5 C-Atomen, wie ein Methyl- oder Ethylrest sein. $R^3$ bedeutet einen Alkylrest mit 1 bis 15 C-Atomen, wie einen Methyl-, Ethyl-, n-Propyl-, i-Propyl-, n-Butyl-, i-Butyl-, tert.-Butyl-, n-Pentyl-, Palmityl- oder Stearylrest, einen cycloaliphatischen Rest mit 5 bis 7 Kohlenstoffatomen, wie einen Cyclopentyl-, Cyclohexyl- oder Cycloheptylrest oder einen aromatischen Rest, wie einen Phenylrest, der durch Alkyl- oder Halogenreste substituiert sein kann. Geeignete 4-Acyloxy-2-butenale sind z.B. 2-Methyl-4-acetoxy-2-butenal, 4-Acetoxy-2-butenal, 3-Methyl-4-acetoxy-2-butenal, 2-Ethyl-4-acetoxy-2-butenal und 3-Ethyl-4-acetoxy-2-butenal.

Als Carbonsäureanhydride der Formel III verwendet man z.B. Essigsäureanhydrid, Propionsäureanhydrid, Benzoesäureanhydrid oder Cyclohexancarbonsäureanhydrid.

Bezogen auf ein Mol des 4-Acyloxy-2-butenals werden weniger als 4 Mol, z.B. 0,5 bis 3,8, vorzugsweise 1 bis 3 Mol des Anhydrids angewendet.

Als entsprechende Carbonsäuren, die somit die Formel $R^3COOH$ aufweisen, verwendet man z.B. Essigsäure, Propionsäure, Benzoesäure oder Cyclohexancarbonsäure. Man wendet die Säuren in einer 0,1- bis 3,1-, insbesondere 0,5- bis 2-molaren Menge, bezogen auf das Acyloxy-2-butenal, an.

Als Ammonium- oder Alkalisalze verwendet man z.B. Ammonium-, Lithium-, Natrium- oder Kalium-Salze der genannten Carbonsäuren, oder die Oxide, Hydroxide, Carbonate oder Hydrogencarbonate der Alkalimetalle. Sie werden in einer 0,1-bis 1,7-, vorzugsweise 0,5- bis 1-molaren Menge, bezogen auf das Acyloxy-2-butenal, angewendet. Die anstelle der Alkalisalze zu verwendenden Pyridinderivate der Formel IV sind z.B. die folgenden Verbindungen: 4-Dimethylaminopyridin, 4-Diethylaminopyridin, 4-Morpholinpyridin, 4-Pyrrolidinopyridin und 4-Piperidinopyridin. Man wendet diese Verbindungen in einer 0,001- bis 1-, vorzugsweise 0,05- bis 0,5-molaren Menge, bezogen auf das Acyloxy-2-butenal, an.

Das erfindungsgemässe Verfahren sei am Beispiel der Umsetzung von 2-Methyl-4-acetoxy-2-butenal mit Acetanhydrid in Gegenwart von Kaliumacetat zu 2-Methyl-1,4-diacetoxy-1,3-butadien durch die folgenden Formeln beschrieben:

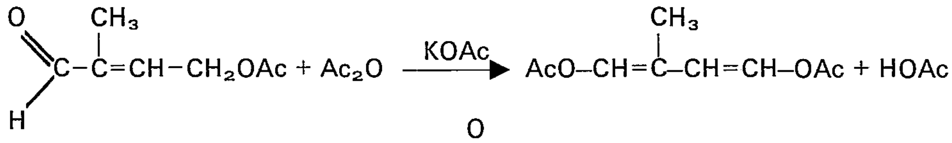


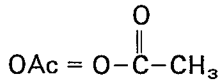

Die Ausgangsstoffe der Formel II lassen sich beispielsweise aus Acetalen des 3-Hydroxy-3-methyl-1-buten-4-als oder aus 3,4-Diacyloxy-1-butenen herstellen (Pure and Applied Chemistry, Band 43, Nr. 1-2, S. 539-544). 4-Acetoxy-2-butenal ist beispielsweise aus 1,1,4-Triacetoxy-2-buten oder 1-Hydroxy-4-acetoxy-2-buten herstellbar [Chem. Ber. *90*, 198 (1957)].

Die Umsetzung führt man bei Temperaturen zwischen 0 und 200°C, insbesondere bei 80 bis 150°C durch. Die Reaktionszeiten betragen z.B. 0,5 bis 100 Stunden. Zum Reaktionsgemisch wird dann Wasser gegeben. Bezogen auf ein Mol des 4-Acyloxy-2-butenals II werden 5 bis 100 Mol, vorzugsweise 15 bis 50 Mol Wasser verwendet. Anschliessend werden organische und wässerige Phase getrennt.

Es ist empfehlenswert, nach der Phasentrennung die abgetrennte wässerige Phase mehrmals mit einem organischen Lösungsmittel zu extrahieren. Hierfür sind solche Lösungsmittel besonders geeignet, die die gebildeten 1,4-Diacyloxy-1,3-butadiene gut, Wasser und die jeweilige Carbonsäure jedoch schlecht lösen. Besonders geeignet sind aliphatische Kohlenwasserstoffe, wie Alkane, Alkene, Alkine, Cycloalkane, aromatische Kohlenwasserstoffe oder Gemische dieser Verbindungen. Das Volumenverhältnis von Wasserphase zum organischen Lösungsmittel hält man zweckmässig zwischen den Grenzen 1:5 bis 5:1. Diese Extrakte können mit der organischen Phase vereinigt werden. Nach dem Abdestillieren des organischen Lösungsmittels aus der organischen Phase wird das 1,4-Diacyloxy-1,3-butadien I durch fraktionierte Destillation gewonnen. Für viele Verwendungszwecke ist die Reinheit des nach dem Abziehen des Lösungsmittels erhaltenen 1,4-Diacyloxy-1,3-butadiens auch ohne Destillation ausreichend. Das Verfahren kann chargenweise oder kontinuierlich, drucklos oder unter Druck durchgeführt werden. Unumgesetzte 4-Acyloxy-2-butenale II können gegebenenfalls destillativ von den entstandenen Wertprodukten I abgetrennt und erneut für die Umsetzung mit Carbonsäureanhydriden III verwendet werden.

Nach dem neuen Verfahren lassen sich die 1,4-Diacyloxy-1,3-butadiene in besonders vorteilhafter Weise und in hoher Ausbeute herstellen. Dies ist überraschend, denn es war nicht vorauszusehen, dass man mit den erfindungsgemässen Molverhältnissen der Einsatzstoffe und bei der wässerigen Aufarbeitung höhere Ausbeuten an den 1,4-Diacyloxy-1,3-butadienen als nach den bekannten Verfahren erzielt. Es war nämlich bekannt, dass man bei der Umsetzung von (E)-2-Methyl-2-butenal (Tiglinaldehyd) mit Acetanhydrid in Gegenwart von Natriumacetat bei Einhaltung von Molverhältnissen (1:1,4:0,85), die den erfindungsgemässen entsprechen, und Aufarbeitung mit Wasser nur Ausbeuten an 1-Acetoxy-2-methyl-1,3-butadien von 10,8% erzielt [Helvetica Chimica Aceta, *44*, 1 (1961)]. Aus der DE-PS 1020621 ist weiterhin bekannt, dass man 1,4-Diacetoxy-1,3-butadien durch Erhitzen von 1,1,4-Triacetoxy-2-buten in Gegenwart von wasserfreiem Natriumacetat und Acetanhydrid und anschliessende Aufarbeitung mit Wasser in nur 57%iger Ausbeute erhält. Aus der DT-AS 2309885 ist zwar bekannt, 2-Alkyl-4-alkoxy-2-butenale mit Acetanhydrid in Gegenwart von Alkalisalzen zu 1-Acetoxy-2-alkyl-4-alkoxy-1,3-butadienen umzusetzen und derartige Reaktionsgemische mit Wasser aufzuarbeiten. Hieraus konnte man jedoch nicht auf das Verhalten der 2-Methyl-1,4-diacyloxy-1,3-butadiene bei der Aufarbeitung mit Wasser schliessen, so dass über das Ausmass der Hydrolyse und die Ausbeuten keine Voraussagen möglich waren.

Die 1,4-Diacyloxy-1,3-butadiene der Formel I sind wertvolle Zwischenprodukte. Sie sind beispielsweise als Dienkomponenten für Diels-Alder-Reaktionen geeignet [J. Org. Chem. *41*, 2625 (1976); Chem. Ber. *90*, 187 (1957); Angew. Chem. *89*, 822 (1977)].

*Beispiel 1:*

In einem 2-Liter-Vierhalskolben mit Rückflusskühler, Rührer und Kontakt-Innenthermometer wurde ein Gemisch aus 450 g Acetanhydrid (4,4 Mol) und 58 g Essigsäure (0,97 Mol) auf 90 bis 100°C erwärmt. Es wurden 3 l Stickstoff pro Stunde in den Gasraum über der Lösung geleitet. Ohne weiteres Heizen wurden nun portionsweise 67 g wasserfreies Kaliumcarbonat (0,49 Mol) innerhalb von 0,5 Stunden eingetragen. Hierbei traten Wärme- und Kohlendioxidentwicklung auf. Die Suspension wurde auf 100°C erwärmt, 0,5 Stunden bei dieser Temperatur gerührt und innerhalb von 4 Minuten bei 110°C mit 284 g 2-Methyl-4-acetoxy-2-butenal (2 Mol) versetzt. Die dabei auf 85°C absinkende Temperatur wurde wieder auf 110°C gebracht. Das Reaktionsgemisch wurde unter Stickstoff 20 Stunden bei 110 ±1°C gerührt. Drei Stunden nach der Aldehydzugabe war aus der Suspension eine klare gelbbraune Lösung geworden. Nach der 20stündigen Reaktionszeit war im Reaktionsgemisch gaschromatographisch kein 2-Methyl-4-acetoxy-2-butenal mehr nachweisbar.

Nach dem Abkühlen des Reaktionsgemisches auf 20 bis 30°C wurde 1 l Wasser zugegeben. Die Temperatur sank auf 14°C. Man rührte im Reaktionskolben eine Stunde bei 15 bis 30°C, wobei man, wenn die Temperatur über 30°C stieg, abkühlte. Die Phasen wurden im Scheidetrichter getrennt. Die organische Phase wurde dann einmal mit 100 ml Wasser ausgeschüttelt. Die organische Phase wurde am Rotationsverdampfer bei 50°C Badtemperatur/15 mbar eingeengt. Man isolierte als Rückstand 357 g eines Öls, aus dem man nach fraktionierter Destillation 316 g 2-Methyl-1,4-diacetoxy-1,3-butadien vom Siedepunkt 78-86°C/0,3 bis 0,6 mbar ($n_D^{20}$=1,4962) erhielt (86% d.Th., bezogen auf eingesetztes 2-Methyl-4-acetoxy-2-butenal).

Durch zweimalige Extraktion der vereinigten Wasserphasen mit je 100 ml Petrolether (Siedepunkt 60-70°C), Vereinigen und Eindampfen der Petroletherextrakte und fraktionierte Destillation des Rückstandes erhielt man zusätzlich 16 g 2-Methyl-1,4-diacetoxy-1,3-butadien (4% d.Th., bezogen auf eingesetztes 2-Methyl-4-acetoxy-2-butenal).

*Beispiel 2:*

Zu einem Gemisch aus 284 g 2-Methyl-4-acetoxy-2-butenal (2 Mol), 459 g Acetanhydrid (4,5 Mol) und 60 g Essigsäure (1 Mol) fügte man bei 20°C 2,44 g 4-Dimethylaminopyridin (0,02 Mol) hinzu. Man erwärmte das Reaktionsgemisch unter strömendem Stickstoff auf 110±2°C, rührte es 70 Stunden lang bei dieser Temperatur und liess es dann auf Raumtemperatur abkühlen.

Nach der in Beispiel 1 beschriebenen Aufarbeitung mit 1 l Wasser und 200 ml Petrolether erhielt man durch fraktionierte Destillation 17 g unumgesetztes 2-Methyl-4-acetoxy-2-butenal (6%), 276 g 2-Methyl-1,4-diacetoxy-1,3-butadien vom Siedepunkt 73-78°C/0,1 mbar ($n_D^{20}$ =1,4950) (75% d.Th., bezogen auf eingesetztes 2-Methyl-4-acetoxy-2-butenal) und 28 g 2-Methyl-1,1,4-triacetoxy-2-buten vom Siedepunkt 100-110°C/0,4 mbar (6%).

Da sich 2-Methyl-1,1,4-triacetoxy-2-buten nach EP 10656 auf einfache Weise zu 2-Methyl-4-acetoxy-2-butenal hydrolysieren lässt, berechnet sich die auf umgesetztes 2-Methyl-4-acetoxy-2-butenal bezogene Ausbeute an 2-Methyl-1,4-diacetoxy-1,3-butadien zu 85%.

## Patentanspruch

Verfahren zur Herstellung von 1,4-Diacyloxy-1,3-butadienen der Formel

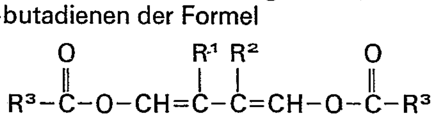 (I)

in der $R^1$ und $R^2$ Wasserstoff oder einer dieser Reste einen Alkylrest mit 1 bis 5 Kohlenstoffatomen und der andere Wasserstoff, und $R^3$ einen Alkylrest mit 1 bis 15 Kohlenstoffatomen, einen cycloaliphatischen Rest mit 5 bis 7 Kohlenstoffatomen oder einen aromatischen Rest bedeuten, durch Umsetzung von 4-Acyloxy-2-butenalen der Formel

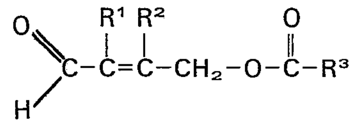 (II)

in der $R^1$, $R^2$ und $R^3$ die obengenannte Bedeutung besitzen, mit Carbonsäureanhydriden der Formel

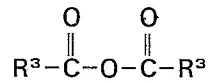 (III)

in Gegenwart der entsprechenden Carbonsäuren, dadurch gekennzeichnet, dass man, bezogen auf 1 Mol des 4-Acyloxy-2-butenals, weniger als 4 Mol des Carbonsäureanhydrids anwendet, die Umsetzung bei Temperaturen zwischen 0 und 200°C und entweder in Gegenwart von Ammonium- oder Alkalisalzen oder von Pyridinderivaten der Formel

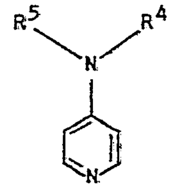 (IV)

in der $R^4$ und $R^5$ gleich oder verschieden sein können und Alkylreste mit 1 bis 5 Kohlenstoffatomen darstellen oder zusammen mit dem N-Atom einen Pyrrolidin-, Piperidin- oder Morpholinrest bedeuten, durchführt, nach der Umsetzung zum Reaktionsgemisch, bezogen auf ein Mol des eingesetzten 4-Acyloxy-2-butenals, 5 bis 100 Mol Wasser gibt und die 1,4-Diacyloxy-1,3-butadiene durch Abtrennung von der Wasserphase isoliert.

## Claim

A process for the preparation of a 1,4-diacyloxy-1,3-butadiene of the formula

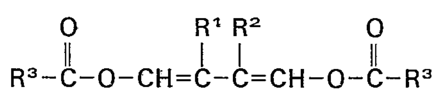 (I)

where $R^1$ and $R^2$ are each hydrogen, or one of these radicals is alkyl of 1 to 5 carbon atoms and the other is hydrogen, and $R^3$ is alkyl of 1 to 15 carbon atoms, a cycloaliphatic radical of 5 to 7 carbon atoms or an aromatic radical, by reacting a 4-acyloxy-2-butenal of the formula

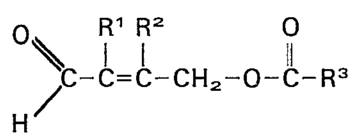 (II)

where $R^1$, $R^2$ and $R^3$ have the above meanings, with a carboxylic acid anhydride of the formula

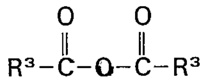

(III)

in the presence of the corresponding carboxylic acid, wherein less than 4 moles of the carboxylic acid anhydride is used per mole of the 4-acyloxy-2-butenal; the reaction is carried out at from 0 to 200°C either in the presence of an ammonium or alkali metal salt or in the presence of a pyridine derivative of the formula

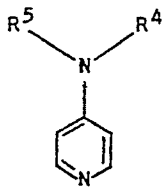

(IV)

where $R^4$ and $R^5$ may be identical or different and are each alkyl of 1 to 5 carbon atoms or, together with the nitrogen atom, denote a pyrrolidine, piperidine or morpholine radical; after the reaction to give the reaction mixture, 5 to 100 moles of water are added per mole of the 4-acyloxy-2-butenal used; and the 1,4-diacyloxy-1,3-butadiene is isolated by separating off the aqueous phase.

## Revendication

Procédé de préparation de diacyl-1,4-butadiènes-1,3 de la formule

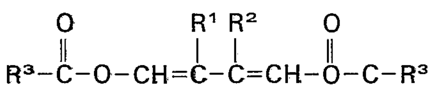

(I)

dans laquelle $R^1$ et $R^2$ désignent des atomes d'hydrogène ou l'un de ces substituants désigne un atome d'hydrogène et l'autre un radical alcoyle en $C_1$ à $C_5$ et $R^3$ désigne un radical alcoyle en $C_1$ à $C_{15}$, un groupe cycloaliphatique en $C_5$ à $C_7$ ou un groupe aromatique, par réaction d'acyloxy-4-buténals-2 de la formule

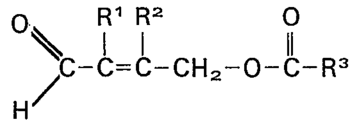

(II)

dans laquelle $R^1$, $R^2$ et $R^3$ possèdent la signification définie, avec des anhydrides d'acides carboxyliques de la formule

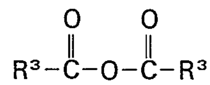

(III)

en présence de l'acide carboxylique correspondant, caractérisé en ce que l'anhydride carboxylique est mis en œuvre en une proportion inférieure à 4 moles par mole de l'acyloxy-4-buténal-2 et la réaction est réalisée entre 0 et 200°C en présence de sels d'ammonium ou de sels de métaux alcalins ou de dérivés de la pyridine de la formule

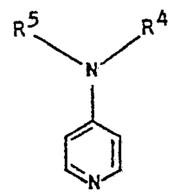

(IV)

dans laquelle $R^4$ et $R^5$, qui peuvent être identiques ou différents, représentent des radicaux alcoyle en $C_2$ à $C_5$ ou forment avec l'atome d'azote un groupe pyrrolidine, pipéridine ou morpholine, la réaction étant suivie de l'addition au mélange réactionnel de 5 à 100 moles d'eau par mole d'acyloxy-4-buténal-2 mis en œuvre et de l'isolement du diacyloxy-1,4-butadiène-1,3 formé par extraction de la phase aqueuse.